# EUROPÄISCHE PATENTSCHRIFT

(11) **EP 0 822 402 B1**
(45) Veröffentlichungstag und Bekanntmachung des Hinweises auf die Patenterteilung: **25.10.2000**
(21) Anmeldenummer: 97113001.8
(22) Anmeldetag: 29.07.1997
(51) Int. Cl.: G01N 1/24, G01N 33/00

(54) **Verfahren zum Aufbereiten eines Messgasstroms**
Method of conditioning a stream of measurement gas
Méthode de conditionnement d'un débit de gaz de mesure

(30) Priorität: 01.08.1996 DE 19631002
(43) Veröffentlichungstag der Anmeldung: 04.02.1998
(73) Patentinhaber: Testo GmbH & Co., 79853 Lenzkirch (DE)
(72) Erfinder: Springmann, Thomas, 79102 Freiburg (DE)
(74) Vertreter: Patentanwälte Westphal, Mussgnug & Partner

(56) Entgegenhaltungen:
- EP-A- 0 194 955
- DE-A- 4 216 404
- US-A- 4 100 806
- US-A- 4 817 441
- US-A- 4 974 453
- US-A- 5 415 025

## Beschreibung

Die Erfindung betrifft ein Verfahren zum Aufbereiten eines Meßgasstroms für eine Gasanalyseeinrichtung gemäß Oberbegriff des Anspruchs 1.

Zur Analyse des Rauchgases von Feuerungsanalgen oder des Abgases von Motoren, wie beispielsweise Gasmotoren, wird ein Meßgasstrom über eine Meßgassonde aus dem Abgasstrom abgezogen und über einen Meßgasschlauch einem Kondensatabscheider zugeführt, bevor die Analyse in der Gasanalyseeinrichtung, welche direkt oder unter Zwischenschaltung einer - auch längeren Zuleitung - dem Kondensatabscheider nachgeordnet ist, vorgenommen werden kann. Für die Gasanalyse ist es erforderlich, das Meßgas aufzubereiten, insbesondere die im Meßgas enthaltende Feuchtigkeit zu entfernen.

Beim Transport des Meßgases von der Sonde zum Kondensatabscheider besteht die Gefahr, daß sich infolge von Abkühleffekten vorzeitig Kondensat an der Innenwandung des Meßgasschlauchs niederschlägt, so daß in der Folge weiteres Meßgas mit dem flüssigen Kondensat in Berührung kommt. Dabei wird beispielsweise NO₂ und SO₂ teilweise im Kondensat gebunden mit der Konsequenz, daß sich die Konzentration dieser Bestandteile beim Durchströmen des Meßgasschlauchs verändern, so daß der von der Analyseeinrichtung ermittelte Meßwert von der im Abgasstrom tatsächlich vorhandenen Konzentration abweicht.

Um derartige Meßfehler zu vermeiden, ist es beispielsweise aus der DE 42 16 404 A1 bekannt, den Meßgasschlauch zu beheizen. Damit ist sichergestellt, daß das Meßgas beim Durchströmen auf einer Temperatur gehalten wird, die oberhalb des Taupunkts liegt. Ein vorzeitiges Abscheiden von Kondensat ist damit verhindert. Die Kondensatabscheidung erfolgt vollständig in dem dem Meßgasschlauch nachgeschalteten Kondensatabscheider. Damit ist sichergestellt, daß das Kondensat schnell und längs einer kurzen Wegstrecke des Gasströmungswegs niederschlagen kann, so daß die Berührungsdauer des Meßgases mit dem flüssigen Kondensat begrenzt ist. NO₂ und SO₂ können nur in einem geringen, das Meßergebnis unerheblich verfälschenden Ausmaß gebunden werden.

Obwohl sich diese Art der Gasaufbereitung prinzipiell bewährt hat, bestehen in der Praxis dennoch Probleme. Diese liegen insbesondere darin begründet, daß ein vergleichsweise hoher technischer Aufwand infolge der Beheizung des Meßgasschlauchs betrieben werden muß. Gravierender wirkt sich der vergleichsweise hohe Energieaufwand aus, da über eine vergleichsweise lange Strecke das Meßgas auf hoher Temperatur gehalten werden muß. Darüber hinaus muß der nachgeschaltete Kondensatabscheider sehr stark gekühlt werden, da im Kondensatraum das Meßgas auf eine Temperatur unterhalb der Taupunkttemperatur abgesenkt werden muß. Da die Abkühlung zur Vermeidung von Meßfehlern auf einer möglichst kurzen Wegstrecke erfolgen soll, ist die einzubringende Kühlleistung sehr hoch. Bei den speziell hier interessierenden mobilen und transportablen Gasanalyseeinrichtungen für Schornsteinfeger, Feuerungstechniker und dergleichen ist dieses Problem um so gravierender, da für die Stromversorgung lediglich Akkumulatoren zur Verfügung stehen. Die Einsatzdauer der Gasanalyseeinrichtung ist deshalb sehr begrenzt und erfordert das Mitführen von Reserveakkus, um die während eines üblichen Arbeitstages anfallenden Meßaufgaben durchführen zu können.

Der Erfindung lag daher das Problem zugrunde, das eingangs beschriebene Verfahren zum Aufbereiten eines Meßgases so zu verbessern, daß es die geschilderten Nachteile nicht mehr aufweist. Insbesondere sollte der für das Aufbereiten erforderliche Energiebedarf gesenkt werden, um längere Betriebszeiten von akkubetriebenen Gasanalyseeinrichtungen zu erhöhen.

Gelöst wird dieses Problem mit einem Verfahren, welches die Merkmale des Anspruchs 1 aufweist.

Vorteilhafte Verfahrensvarianten sind durch die Merkmale der Unteransprüche angegeben.

Die Erfindung basiert auf der Idee, auf eine Beheizung des Meßgasschlauchs vollständig zu verzichten und eine Absorption von beispielsweise NO₂ und SO₂ dadurch weitgehend zu verhindern, daß durch eine Steigerung der Durchflußgeschwindigkeit des Meßgases dessen Verweildauer im Meßgasschlauch erheblich reduziert wird. Dieses Konzept weist gegenüber den bisher bekannten Bemühungen der Fachwelt in die genau entgegengesetzte Richtung. So war bislang versucht worden, die für die Beheizung des Meßgasschlauchs erforderliche Energie dadurch zu reduzieren, daß der Meßgasschlauch mit einem Isoliermantel versehen wird, um zumindest die Wärmeabgabe nach außen hin an die Umgebung zu minimieren. Dies führte zu teilweise unhandlichen und schweren Meßgasschläuchen. Im Gegensatz hierzu gestattet das erfindungsgemäße Verfahren die Verwendung eines dünnen Meßgasschlauchs, der keinerlei Zusatzelemente, wie beispielsweise eine Isolation oder dergleichen benötigt. Als zusätzlicher energieeinsparender Effekt ergibt sich, daß der Meßgasschlauch als Kühlstrecke für das hindurchgeführte Meßgas dient, so daß bei einer entsprechenden Auslegung am Ende des Meßgasschlauchs bereits eine Temperatur erreicht ist, die im wesentlichen der Umgebungstemperatur entspricht. Damit reduziert sich auch die für die restliche Abkühlung des Meßgases auf eine Temperatur unterhalb der Taupunkttemperatur erforderliche Kühlleistung, die von dem in dem Kondensatabscheider integrierten Kühlelement eingebracht werden muß. Gegenüber der bisherigen Situation, bei der eine Temperaturabsenkung im Kondensatabscheider um 200° bis 300° C erreicht werden mußte, ist nunmehr lediglich eine Temperaturabsenkung um ca. 20° C vonnöten. Diese vergleichsweise geringe Kühlleistung kann auch auf erheblich reduzierter Wegstrecke eingebracht werden mit der Konsequenz, daß der Kondensatabscheider erheblich verkleinert werden kann.

Konkret wird der geschilderte Effekt dann erreicht, wenn die mittlere, d. h. über dem Strömungsquerschnitt gemittelte Strömungsgeschwindigkeit des Meßgasstroms einen Wert von zumindest 1,5 m/s annimmt. Optimale Ergebnisse werden dann erzielt, wenn die Strömungsgeschwindigkeit auf einen Wert im Bereich zwischen 4,0 m/s und 7,0 m/s eingestellt wird. Eine Steigerung über den letztgenannten Wert hinaus erscheint wenig sinnvoll, da eine weitere Reduzierung der Absorption von NO₂ und SO₂ das Meßergebnis nicht mehr signifikant beeinflußt, wohingegen der Strömungswiderstand erheblich ansteigt mit der Konsequenz, daß eine Förderpumpe mit erhöhter Leistungsaufnahme erforderlich wird.

Die vorstehend genannten Bedingungen für die Strömungsgeschwindigkeit stellen darüber hinaus sicher, daß an der Schlauchwandung niedergeschlagene Kondensattröpfchen dort nicht anhaften können, sondern vom Meßgasstrom mitgerissen und zum Kondensatraum des Kondensatabscheiders hin abtransportiert werden.

Für die meisten der in der Praxis vorkommenden Meßaufgaben läßt sich der durch die Absorption von NO₂ und SO₂ bedingte Meßfehler auf vernachlässigbare Werte drücken, wenn die mittlere Verweildauer des Meßgasstroms im Meßgasschlauch weniger als 3 sec. beträgt. Ein Meßfehler von weniger als einem Prozent läßt sich dann erreichen, wenn gemäß einer bevorzugten Verfahrensvariante die Verweildauer im Bereich von 0,25 sec. bis 1,0 sec. beträgt.

Besondere Vorteile ergeben sich bei der Verwendung eines Meßgasschlauchs, der aus PTFE gefertigt ist. Dieses Material ist einerseits ausreichend resistent gegenüber aggressiven Bestandteilen des Meßgases, andererseits besitzt es eine Oberflächenstruktur, die ein Anhaften von Kondensattröpfchen erschwert, so daß diese besonders leicht vom Meßgasstrom mitgerissen werden.

Bevorzugt wird ein Meßgasschlauch mit einem Innendurchmesser im Bereich von 1,5 mm bis 4,0 mm verwendet, ein Innendurchmesser von 2,0 mm hat sich als optimal erwiesen. Die vorstehenden Durchmesserangaben sind abgestimmt auf die eingangs beschriebenen Meßaufgaben aus dem Bereich der Abgastechnik und berücksichtigen darüber hinaus die üblicherweise installierte Förderleistung der Meßgaspumpe, die den Meßgasstrom aus dem Abgasstrom absaugt. Diese Leistung gestattet ein Fördervolumen von ca. 0,9 l/min. Unter Berücksichtigung der durchschnittlich anzutreffenden Zustandsdaten des Meßgases sowie der Strömungswiderstände ergibt sich dann die eingangs beschriebene Geschwindigkeitsbedingung für den Meßgasstrom. Ein Durchmesser von weniger als 1,0 mm führt in der Praxis zu so großen Strömungswiderständen, daß die installierte Pumpenleistung nicht mehr ausreicht. Bei einem Innendurchmesser von mehr als 4,0 mm ist hingegen die Strömungsgeschwindigkeit im Meßgasschlauch bereits so weit abgesunken, daß sich Meßfehler infolge erhöhter Absorption von NO₂ und SO₂ in meßrelevantem Umfang einstellt.

Hinsichtlich der Länge des verwendeten Meßgasschlauches haben sich Werte zwischen 1,5 m und 5,0 m als zweckmäßig erwiesen. Sie kann nach Bedarf variiert werden, um die Endtemperatur am Übergang zum Kondensatabscheider einzustellen. Üblicherweise reicht eine Länge von ca. 3 m aus, um für übliche Meßaufgaben eine Abkühlung auf Umgebungstemperatur sicherzustellen. In diesem Zusammenhang spielen natürlich auch material- und geometriespezifische Gegebenheiten des Schlauches eine wichtige Rolle, da diese den Wärmeübergang über die Schlauchwandung hinweg zur Umgebung hin vorgeben.

Eine ganz besonders bevorzugte Verfahrensvariante sieht vor, einen Kondensatabscheider mit einem integrierten Peltier-Element zu verwenden, wobei die Kaltseite des Peltier-Elements unmittelbar vom Meßgasstrom umströmt wird. Ein solcher Kondensatabscheider ist in der am selben Anmeldetag eingereichten Patentanmeldung No. (tes200 "Kondensatabscheider") beschrieben, auf die insoweit ausdrücklich Bezug genommen wird. Ein derartiger Kondensatabscheider besitzt einen gegenüber bisher bekannten Kondensatabscheidern erheblich besseren Wirkungsgrad, so daß sich der Gesamtenergiebedarf für die Aufbereitung des Meßgases in außergewöhnlich hohem Maße reduzieren läßt.

Die Erfindung wird nachstehend näher anhand von in den Figuren dargestellten Diagrammen erläutert, die als Auswertung von Optimierungsversuchen gewonnen wurden. Es zeigen:
- Figur 1: Absorption von NO₂ in Abhängigkeit verschiedener Innendurchmesser und
- Figur 2: Absorption von SO₂ bei unterschiedlichen Innendurchmessern.

In Reihenversuchen wurde die Absorption von NO₂ und SO₂ in einem unbeheizten Meßgasschlauch aus PTFE untersucht. Bei konstant gehaltener Schlauchlänge von zwei Metern wurden die in der nachstehenden Tabelle wiedergegebenen Innendurchmesservariationen durchgeführt und die jeweils erfolgte Absorption von NO₂ und SO₂ bestimmt. Es wurde Prüfgas mit einer Konzentration von 179 ppm NO₂ und 481 ppm SO₂ verwendet, welches mit einer Wasservorlage von 60 Grad Celsius angefeuchtet wurde. Der Eingangstaupunkt betrug 60 Grad Celsius, der Ausgangstaupunkt 23 Grad Celsius (Umgebungsbedingung). Der Durchfluß wurde auf konstant 0,9 Liter pro Minute eingestellt.

**Tabelle**

| Innendurchmesser (mm) | NO2-Absorption (%) | SO2-Absorption (%) |
|---|---|---|
| 1,5 | 0,2 | 0,2 |
| 2 | 0,4 | 0,3 |
| 3 | 2,6 | 0,4 |
| 4 | 4,1 | 1,3 |
| 6 | 6 | 2 |

Die aus obiger Tabelle entnehmbaren Werte wurden in Form von Diagrammen dargestellt.

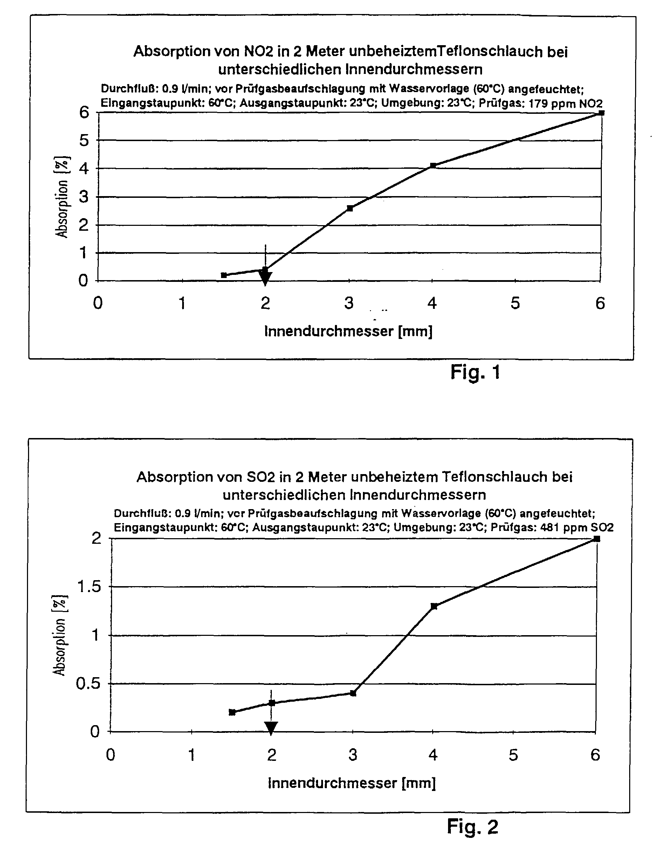

Das in Figur 1 dargestellte Diagramm zeigt die Absorption von NO₂ in Abhängigkeit des Innendurchmessers. Nach einem zunächst flachen Anstieg der Absorption von 0,2 % auf 0,4 % beim Übergang von einem Innendurchmesser von 1,5 mm auf 2 mm steigt der Kurvenverlauf relativ steil an und schwächt sich erst wieder in Richtung großer Durchmesserwerte ab, bis er schließlich einen Wert von 6 % bei einem Durchmesser von 6 mm erreicht.

Aufgrund dieses Kurvenverlaufs ist zu folgern, daß ein Innendurchmesser von 2 mm unter den gegebenen Meßbedingungen optimal ist, da einerseits die Absorption noch gering ist, andererseits der Schirmungswiderstand noch nicht all zu ausgeprägt sein sollte.

Der größte der angegebenen Durchmesser (6 mm) entspricht dem Durchmesser der bisher verwendeten Meßgasleitungen und verdeutlicht das Verbesserungspotential des erfindungsgemäßen Verfahrens. Dieser Meßpunkt verdeutlicht auch, daß bei Meßschläuchen mit derzeit üblichen Durchmessern eine Beheizung unumgänglich ist, da eine Absorption in Höhe von 6 % nicht hinnehmbar ist.

Das in Figur 2 dargestellte Diagramm zeigt die Absorption von SO₂ in Abhängigkeit der oben genannten Durchmesser. Der Kurvenverlauf zeigt im Bereich von 1,5 mm bis 3 mm einen flachen Verlauf mit Absorptionswerten zwischen 0,2 % und 0,4 %. Zu größeren Durchmessern hin steigt die Kurve sehr stark an und erreicht bei dem größten Durchmesser eine Absorption von 2 %. Hiernach wäre abzuleiten, daß im Hinblick auf die Absorption von SO₂ Innendurchmesser bis etwa 3 mm günstig sind. Unter der zusätzlichen Berücksichtigung des Absorbtionsverhaltens von NO₂ kann abgeleitet werden, daß ein Innendurchmesser von ca. 2 mm das Optimum darstellt.

Die Versuche ergaben die Bestätigung, daß ein derartig dimensionierter Schlauch geeignet ist, die Anlagerung von Kondensattröpfchen an der Innenwandung sicher zu vermeiden. Eine Ansammlung solcher Tröpfchen zum sogenannten Wassersack, durch den das Meßgas hindurchgeleitet werden müßte, kann nicht mehr entstehen. Der Meßgasschlauch wird damit gleichzeitig lageunabhängig, d.h. er kann in an sich beliebiger Anordnung, beispielsweise also auch durchhängend oder in Überkopfanordnung, eingesetzt werden.

Weiterhin konnte durch die Versuche bestätigt werden, daß der getestete Meßgasschlauch wenig schmutzempfindlich ist. Während eines über zwei Tage hinweg geführten Dauerversuchs war eine zwischenzeitliche Reinigung nicht erforderlich. Damit zeigt sich die Praxistauglichkeit des erfindungsgemäßen Konzepts.

## Patentansprüche

1. Verfahren zum Aufbereiten eines Meßgasstroms für eine Gasanalyseeinrichtung, bei dem Meßgas über eine Meßgassonde aus einem Abgasstrom abgezogen, über einen Meßgasschlauch einem Kondensatabscheider zugeführt und abschließend in der Gasanalyseeinrichtung hinsichtlich bestimmter Bestandteile, wie beispielsweise NO₂ und SO₂, analysiert wird, dadurch gekennzeichnet, daß die mittlere Strömungsgeschwindigkeit des Meßgasstroms im Meßgasschlauch auf einen Wert von wenigstens 1,5 m/s eingestellt wird.

2. Verfahren nach Anspruch 1, gekennzeichnet durch eine Strömungsgeschwindigkeit im Bereich zwischen 4,0 m/s und 7,0 m/s.

3. Verfahren nach Anspruch 1 oder 2, gekennzeichnet durch eine mittlere Verweildauer des Meßgasstroms im Meßgasschlauch von weniger als 3,0 sec.

4. Verfahren nach Anspruch 3, gekennzeichnet durch eine Verweildauer im Bereich zwischen 0,25 sec. und 1,0 sec.

5. Verfahren nach einem der vorstehenden Ansprüche, gekennzeichnet durch die Verwendung eines Meßgasschlauchs aus PTFE.

6. Verfahren nach einem der vorstehenden Ansprüche, gekennzeichnet durch die Verwendung eines Meßgasschlauchs mit einem Innendurchmesser im Bereich von 1,0 mm bis 4,0 mm, vorzugsweise mit einem Innendurchmesser von 2,0 mm.

7. Verfahren nach einem der vorstehenden Ansprüche, gekennzeichnet durch die Verwendung eines Meßgasschlauchs mit einer Länge im Bereich zwischen 1,5 m und 5,0 m.

8. Verfahren nach einem der vorstehenden Ansprüche, gekennzeichnet durch die Verwendung eines Kondensatabscheiders, der mit einem integrierten Peltier-Element gekühlt wird, wobei die Kaltseite es Peltier-Elements unmittelbar vom Meßgasstrom umströmt wird.

## Claims

1. A process for preparing a stream of measuring gas for a gas analysis apparatus, in which measuring gas is removed via a measuring gas probe from a stream of exhaust gas, is supplied via a measuring gas flexible tube to a condensate separator and finally is analysed in the gas analysis apparatus with respect to specific constituents, such as for example NO₂ and SO₂,
**characterised in that** the mean flow velocity of the stream of measuring gas in the measuring gas flexible tube is set at a value of at least 1.5 m/s.

2. A process according to Claim 1,
**characterised by** a flow velocity in the range between 4.0 m/s and 7.0 m/s.

3. A process according to Claim 1 or 2,
**characterised by** a mean sojourn time of the stream of measuring gas in the flexible tube for the measuring gas of less than 3.0 sec.

4. A process according to Claim 3,
**characterised by** a sojourn time in the range between 0.25 sec. and 1.0 sec.

5. A process according to one of the preceding Claims,
**characterised by** the use of a flexible tube for the measuring gas that is made from PTFE.

6. A process according to one of the preceding Claims,
**characterised by** the use of a flexible tube for the measuring gas having an internal diameter in the range from 1.0 mm to 4.0 mm, preferably with an internal diameter of 2.0 mm.

7. A process according to one of the preceding Claims,
**characterised by** the use of a flexible tube for the measuring gas having a length in the range between 1.5 m and 5.0 m.

8. A process according to one of the preceding Claims,
**characterised by** the use of a condensate separator, which is cooled with an integrated Peltier element, the stream of measuring gas flowing directly around the cold side of the Peltier element.

## Revendications

1. Procédé de conditionnement d'un courant de gaz de mesure pour dispositif d'analyse de gaz, dans lequel on prélève le gaz de mesure dans un courant de gaz d'échappement par l'intermédiaire d'une sonde de gaz de mesure, on le conduit par un tube de gaz de mesure dans un séparateur de condensats puis on l'analyse dans le dispositif d'analyse de gaz quant à ses composants, comme par exemple NO₂ et SO₂,
caractérisé en ce que
la vitesse moyenne d'écoulement du courant de gaz de mesure dans le tube de gaz de mesure est réglée à une valeur d'au moins 1,5 m/seconde.

2. Procédé selon la revendication 1,
caractérisé par
une vitesse d'écoulement comprise entre 4,0 m/s et 7,0 m/s.

3. Procédé selon la revendication 1 ou 2,
caractérisé par
un temps de séjour moyen du courant de gaz de mesure dans le tube de gaz de mesure inférieur à 3,0 secondes.

4. Procédé selon la revendication 3,
caractérisé par
un temps de séjour compris entre 0,25 seconde et 1,0 seconde.

5. Procédé selon l'une des revendications précédentes,
caractérisé par
l'utilisation d'un tube de gaz de mesure en PTFE.

6. Procédé selon l'une des revendications précédentes,
caractérisé par
l'utilisation d'un tube de gaz de mesure ayant un diamètre interne compris entre 1,0 mm et 4,0 mm, de préférence ayant un diamètre interne de 2,0 mm.

7. Procédé selon l'une des revendications précédentes,
caractérisé par
l'utilisation d'un tube de gaz de mesure ayant une longueur comprise entre 1,5 mètre et 5,0 mètres.

8. Procédé selon l'une des revendications précédentes,
caractérisé par
l'utilisation d'un séparateur de condensat refroidi par un élément de Peltier intégré, le côté froid de l'élément de Peltier étant immédiatement environné par le courant de gaz de mesure.
